# EUROPEAN PATENT APPLICATION

(11) **EP 4 585 076 A1**
(43) Date of publication of application: **16.07.2025**
(21) Application number: 23861947.2
(22) Date of filing: 13.06.2023
(51) Int. Cl.: A24F 40/40, A24F 40/46, A61M 11/00

(54) **AEROSOL GENERATING PRODUCT AND AEROSOL GENERATING SYSTEM**

(30) Priority: 08.09.2022 CN 202222399191 U
(71) Applicant: Shenzhen Merit Technology Co., Ltd., Shenzhen, Guangdong 518105 (CN)
(72) Inventor: YUAN, Tao, Shenzhen, Guangdong 518105 (CN); LIU, Gang, Shenzhen, Guangdong 518105 (CN); LIN, Zhiwen, Shenzhen, Guangdong 518105 (CN); LI, Tao, Shenzhen, Guangdong 518105 (CN); LIU, Yujie, Shenzhen, Guangdong 518105 (CN); TANG, Jianguo, Shenzhen, Guangdong 518105 (CN); LIANG, Feng, Shenzhen, Guangdong 518105 (CN)
(74) Representative: Herrero & Asociados, S.L.
(86) International application number: PCT/CN2023/099971
(87) International publication number: WO 2024/051244

(57) **Abstract**

The present application provides an aerosol generating product and an aerosol generating system. The aerosol generating product comprises a matrix section, a cooling section and a filtering piece. Specifically, the matrix section is provided with an aerosol generating matrix; the cooling section is located at one end of the matrix section and has a first airflow channel; and the filtering piece is located in the first airflow channel. The filtering piece has a second airflow channel, and/or the filtering piece cooperates with a side wall of the first airflow channel to form the second airflow channel, wherein the second airflow channel is in fluid communication with the first airflow channel. The aerosol generating product provided by the the present application has a two-section structure, and thus has a simpler structure and low processing costs; and the filtering piece is arranged in the first airflow channel, and has a relatively small size, and thus the adsorption effect on aerosol can be reduced, such that the amount of aerosol available for a user is increased.

## Description

### CROSS REFERENCE TO RELATED APPLICATIONS

The present application claims the priority of Chinese patent application No. 202222399191.6, filed on September 08, 2022, and the entire contents of which are hereby incorporated by reference in its entirety.

### TECHNICAL FIELD

The present disclosure relates to the field of atomizing technologies, and in particular to an aerosol-generating product and an aerosol-generating system.

### BACKGROUND

An aerosol-generating system generally includes an aerosol-generating device and an aerosol-generating product. The aerosol-generating device is configured to contain the aerosol-generating product and heat the aerosol-generating product to generate an aerosol. The aerosol-generating system can be applied in different fields, such as medical atomization, beauty atomization, recreational inhalation, etc.

In a usage scenario, the aerosol-generating product includes a solid substrate of plant leaves containing a specific aroma, which is baked in a heat-not-burning manner, such that the solid substrate of plant leaves generates an aerosol for use by a user.

However, a conventional aerosol-generating product is generally of a three-stage or even more-stage structure including a substrate section, a cooling section, and a filtering section, which is relatively complex and can only generate a small amount of aerosol for use by the user.

### SUMMARY OF THE DISCLOSURE

The present disclosure provides an aerosol-generating product and an aerosol-generating system that can solve the problems of the existing aerosol-generating product's complex structure and the small amount of aerosol available to the user.

To solve the above technical problems, the present disclosure provides an aerosol-generating product, including: a substrate section, arranged with an aerosol-generating substrate; a cooling section, disposed on an end of the substrate section and including a first airflow channel; and a filtering member, disposed in the first airflow channel; wherein the filtering member includes a second airflow channel; and/or, the filtering member cooperates with the side wall of the first airflow channel to form a second airflow channel; the second airflow channel is in fluid communication with the first airflow channel.

In some embodiments, the outer surface of the filtering member defines a plurality of first grooves, and the plurality of first grooves cooperate with the inner wall surface of the first airflow channel to form the second airflow channel; or, the inner wall surface of the first airflow channel defines a plurality of second grooves facing the filtering member, and the plurality of second grooves cooperate with the outer surface of the filtering member to form the second airflow channel; or, the outer surface of the filtering member defines a plurality of first grooves, and the inner wall surface of the first airflow channel defines a plurality of second grooves; the plurality of first grooves cooperate with the plurality of second grooves to form the second airflow channel.

In some embodiments, each first groove and/or each second groove is arranged in a straight-line extension or in a curved extension along the axial direction of the aerosol-generating product.

In some embodiments, the distance between the substrate section and the filtering member is in a range of 10 mm-40 mm.

In some embodiments, the end of the filtering member away from the substrate section is flush with the end of the cooling section away from the substrate section.

In some embodiments, the aerosol-generating product further includes: a casing, arranged on the outer surfaces of the substrate section and the cooling section.

In some embodiments, the outer surface of the cooling section defines a plurality of third grooves; the casing cooperates with the plurality of third grooves to form a third airflow channel; the casing defines a plurality of first through holes facing and in communication with the plurality of third grooves, and the plurality of first through holes are configured to guide outside air into the third airflow channel.

In some embodiments, each third groove is arranged in a straight-line extension or in a curved extension along the axial direction of the aerosol-generating product.

In some embodiments, the end of the third airflow channel away from the substrate section is an open end; the outside air is capable of entering the third airflow channel through the plurality of first through holes and flowing out of the open end.

In some embodiments, each third groove extends to the end of the cooling section close to the substrate section; the outside air is capable of entering the third airflow channel through the plurality of first through holes, then entering the substrate section connected to the third airflow channel, and flowing out through the first airflow channel and the second airflow channel.

In some embodiments, the casing includes sidewalls arranged on side surfaces of the substrate section and the cooling section, and a bottom wall arranged on the end of the substrate section away from the cooling section; the bottom wall defining a second through hole.

To solve the above technical problems, the present disclosure further provides an aerosol-generating system, including: the aerosol-generating product as above; and an aerosol-generating device, including a receiving cavity and a heating component; wherein the receiving cavity is configured to contain the aerosol-generating product, and the heating component is configured to heat the aerosol-generating product to generate an aerosol.

Different from the related art, in the aerosol-generating product and the aerosol-generating system provided by the present disclosure, the aerosol-generating product has a two-stage structure including a substrate section and a cooling section arranged in an axial direction, and a filtering member arranged in the first airflow channel of the cooling section. The filtering member includes a second airflow channel, and/or the second airflow channel is formed by the filtering member and the side wall of the first airflow channel, the second airflow channel being fluid communication with the first airflow channel. Compared to conventional three-stage or four-stage aerosol-generating products, the aerosol-generating product provided by the present disclosure has a simpler structure and lower processing cost. In addition, the filtering member is arranged in the first airflow channel, and the filtering member has a smaller size, which may reduce the adsorption effect on the aerosol and thereby increase the amount of aerosol available to the user.

### BRIEF DESCRIPTION OF THE DRAWINGS

In order to more clearly illustrate the technical solutions in the embodiments of the present disclosure, the accompanying drawings to be used in the description of the embodiments will be briefly introduced below, and it will be obvious that the accompanying drawings in the following description are only some of the embodiments of the present disclosure, and that for those skilled in the art, other accompanying drawings can be obtained based on these drawings without putting forth creative labor.
FIG. 1 is an axial cross-sectional view of an aerosol-generating product in the related art.
FIG. 2 is an axial cross-sectional view of an aerosol-generating product according to some embodiments of the present disclosure.
FIG. 3 is an axial cross-sectional view of an aerosol-generating product according to other embodiments of the present disclosure.
FIG. 4 is a cross-sectional view along line A-A of the aerosol-generating product shown in FIG. 2 according to some embodiments of the present disclosure.
FIG. 5 is a cross-sectional view along line A-A of the aerosol-generating product shown in FIG. 2 according to other embodiments of the present disclosure.
FIG. 6 is an axial cross-sectional view of an aerosol-generating product according to further other embodiments of the present disclosure.
FIG. 7 is an axial cross-sectional view of an aerosol-generating product according to still other embodiments of the present disclosure.
FIG. 8 is a cross-sectional view along line B-B of the aerosol-generating product shown in FIG. 6.
FIG. 9 is a structural schematic view of an aerosol-generating system according to some embodiments of the present disclosure.

### DETAILED DESCRIPTION

The technical solutions in the embodiments of the present disclosure will be described clearly and completely in the following in conjunction with the accompanying drawings, and it is obvious that the described embodiments are only a part of the embodiments of the present disclosure, and not all of the embodiments. Based on the embodiments in the present disclosure, all other embodiments obtained by those skilled in the art without making creative labour fall within the scope of the present disclosure.

The terms "first", "second", and "third" in the present disclosure are intended for descriptive purposes only and are not to be construed as indicating or implying relative importance or implicitly specifying the number of technical features indicated. Thus, a feature defined with "first", "second", "third" may expressly or implicitly include at least one of the features. In the description of the present disclosure, "plurality" means at least two, e.g., two, three, etc., unless otherwise expressly and specifically limited. All directional indications (e.g. up, down, left, right, forward, back ......) in the embodiments of the present disclosure are intended only to explain the relative positional relationships, movements, etc. between components in a particular attitude (as shown in the drawings). When the particular attitude changes, the directional indications also change accordingly. The terms "including" and "having" in the embodiments of the present disclosure, and any variations thereof, are intended to cover non-exclusive inclusion. For example, a process, method, system, product, or apparatus including a series of steps or units is not limited to the listed steps or units, but optionally further includes steps or units that are not listed, or optionally includes other steps or components that are inherent to the process, method, product, or apparatus.

Reference to "embodiments" herein implies that particular features, structures, or characteristics described in conjunction with the embodiments may be included in at least one embodiment of the present disclosure. The occurrence of the described phrases at various points in the specification does not necessarily all refer to the same embodiments, nor are they independent or alternative embodiments that are mutually exclusive of other embodiments. It is understood by those skilled in the art, both explicitly and implicitly, that the embodiments described herein may be combined with other embodiments.

The present disclosure is described in detail below in connection with the accompanying drawings and embodiments.

When the aerosol-generating product is baked at low temperatures in a heat-not-burning manner, the solid substrate of plant leaves with a specific aroma can generate an aerosol with a specific aroma. Since the aerosol-generating product is heated at low temperatures, generally not higher than 350°C, the release of harmful substances in the aerosol can be greatly reduced, with the harmful substances being reduced by more than 90%, which provides consumers with an effective and healthy way to relax.

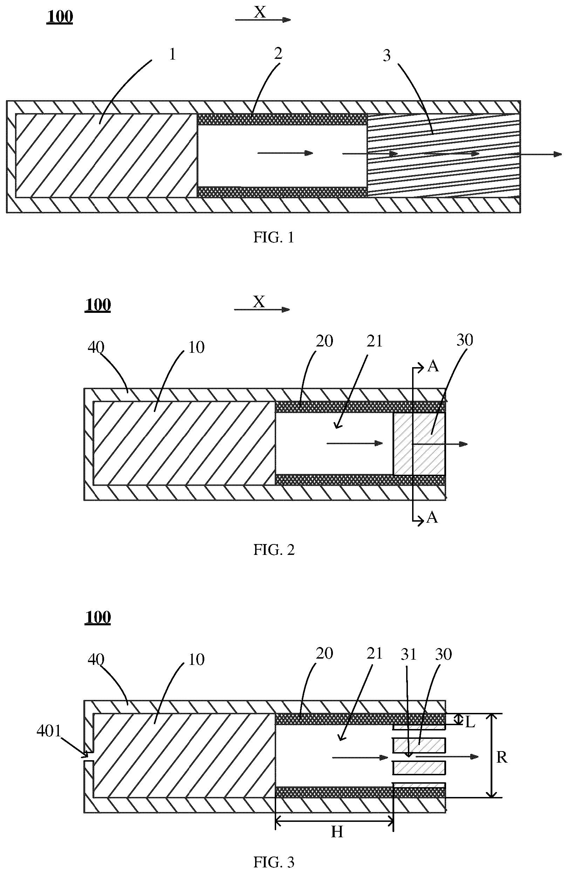

Conventionally, the aerosol-generating product in the related art is generally of a three-stage or four-stage structure in an axial X direction, as shown in FIG. 1. FIG. 1 is an axial cross-sectional view of an aerosol-generating product in the related art. The aerosol-generating product 100 is of a three-stage structure, including a substrate section 1, a cooling section 2, and a filtering section 3. Specifically, the substrate section 1 is arranged with an aerosol-generating substrate for generating an aerosol under a heating condition; the cooling section 2 is configured to cool the aerosol generated by the substrate section 1 to a temperature range suitable for a user to inhale; and the filtering section 3 is configured to filter impurities in the aerosol for the user to inhale.

However, Applicant of the present disclosure found that the conventional aerosol-generating product 100 can only generate a small amount of aerosol available for use by the user, and the conventional aerosol-generating product 100 has a complex structure, which makes the user experience unpleasant.

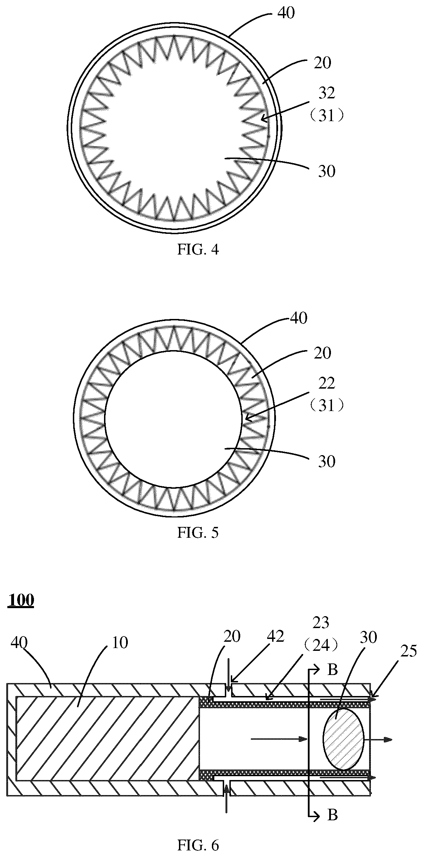

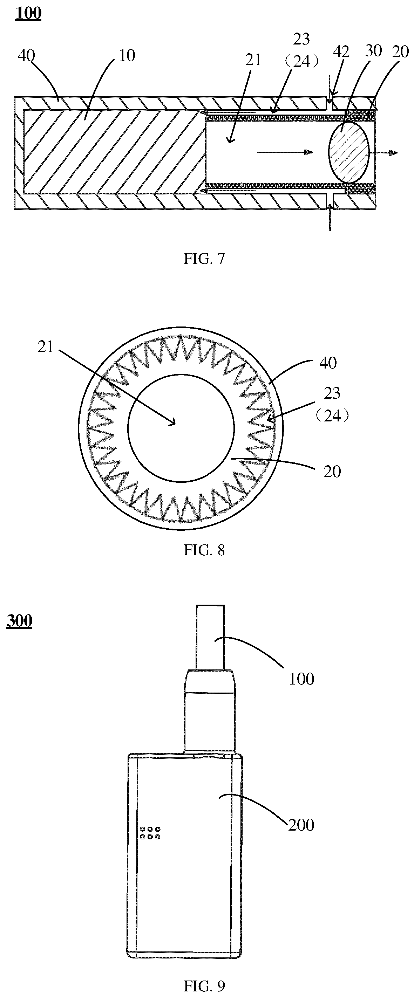

For this reason, referring to FIGS. 2-8, FIG. 2 is an axial cross-sectional view of an aerosol-generating product according to some embodiments of the present disclosure, FIG. 3 is an axial cross-sectional view of an aerosol-generating product according to other embodiments of the present disclosure, FIG. 4 is a cross-sectional view along line A-A of the aerosol-generating product shown in FIG. 2 according to some embodiments of the present disclosure, FIG. 5 is a cross-sectional view along line A-A of the aerosol-generating product shown in FIG. 2 according to other embodiments of the present disclosure, FIG. 6 is an axial cross-sectional view of an aerosol-generating product according to further other embodiments of the present disclosure, FIG. 7 is an axial cross-sectional view of an aerosol-generating product according to still other embodiments of the present disclosure, and FIG. 8 is a cross-sectional view along line B-B of the aerosol-generating product shown in FIG. 6.

The present disclosure provides an aerosol-generating product 100 including a substrate section 10, a cooling section 20, and a filtering member 30 disposed in the cooling section 20.

Specifically, the substrate section 10 is arranged with an aerosol-generating substrate for generating an aerosol. The aerosol-generating substrate may be a solid substrate of plant leaves in the form of filaments, sheets, flakes, granules, powders, columns, etc. containing a specific aroma; or be other types of ordered solid aerosol-generating substrate, disordered solid aerosol-generating substrate, and particulate solid aerosol-generating substrate, etc., as long as it can generate an aerosol that meets the user's needs under a heating condition.

Specifically, the cooling section 20 is disposed on an end of the substrate section 10. The cooling section 20 includes a first airflow channel 21, an end of the first airflow channel 21 being in fluid communication with the substrate section 10, and the other end of the first airflow channel 21 being in communication with outside for guiding out the aerosol generated by the substrate section 10. The material of the cooling section 20 may be a heat transfer material such as cellulose acetate, polylactic acid, resin, silica gel, or vegetable polysaccharide, and is configured to cool the aerosol flowing through the first airflow channel 21 to a suitable temperature range for the user.

The cross-section of the first airflow channel 21 may be of various shapes, such as circular, rectangular, zigzag, pentagonal, elliptical, honeycomb, etc., which is not limited herein, as long as it can have a guiding effect on the aerosol.

The filtering member 30 is disposed in first airflow channel 21, and at least a portion of an outer surface of the filtering member 30 is connected to a side wall of the first airflow channel 21 to fix the filtering member 30. The filtering member 30 is configured to filter the aerosol flowing out from the first airflow channel 21, thereby improving the purity and comfort of the aerosol.

The filtering member 30 may be made of cellulose acetate, polypropylene, paper filter rods, regenerated aerosol-generating substrate, polylactic acid, resin, vegetable polysaccharide, silica gel, ceramics, etc.

The filtering member 30 may be mounted in a designated position in the first airflow channel 21 in a loading manner, after being formed in the cooling section 20. Alternatively, the filtering member 30 may be formed directly with the cooling section 20 by mould opening, which is not limited herein.

Specifically, the filtering member 30 is arranged in the first airflow channel 21, and when the aerosol in the first airflow channel 21 passes through the filtering member 30, the filtering member 30 further has a baffling effect on the aerosol, so as to disperse the aerosol with a vector in the first airflow channel 21, thereby increasing the contact between the aerosol in the first airflow channel 21 and the cooling section 20, and thus improving the cooling effect of the cooling section 20 on the aerosol.

In particular, referring to FIGS. 2 and 7, the shape of the filtering member 30 may be cylindrical, spherical, or polyhedral (not shown). Preferably, the shape of the cross-section of the filtering member 30 is substantially the same as the shape of the cross-section of the first airflow channel 21 in a radial direction perpendicular to the axial direction X, so as to be better fixed to the side wall of the first airflow channel 21, thereby increasing the filtration and the turbulence effect on the aerosol in the first airflow channel 21, and thus improving the user experience.

The aerosol-generating product 100 further includes a casing 40 arranged on an outer surface of the substrate section 10 and the cooling section 20 to encapsulate and protect the substrate section 10 and the cooling section 20. The casing 40 may be made of one or more of paper, a paper tube, tin foil, or aluminium foil. In some embodiments, the aerosol-generating substrate is first wrapped by a rod forming machine to form the substrate section 10, and then the substrate section 10 and the cooling section 20 are cut to a corresponding length on a composite machine and wrapped together by the casing 40 to form the finished aerosol-generating product 100.

Specifically, the casing 40 includes sidewalls arranged on side surfaces of the substrate section 10 and the cooling section 20, and a bottom wall arranged on an end of the substrate section 10 away from the cooling section 20. The bottom wall of the casing 40 may be vented or sealed, which is not limited herein.

In some embodiments, referring to FIG. 2, the bottom wall of the casing 40 is sealed. In this way, when the user inhales, the aerosol-generating product 100 is not affected by external airflow, the cracking reaction of the aerosol-generating substrate is stable, and the inhalation consistence is good. In addition, under a low oxygen condition, the baking temperature can be further increased to enhance the fragrance of the aerosol-generating substrate. Furthermore, the residues and oily substances of the solid aerosol-generating substrate will not rise with the air and contaminate the appliance, which makes it easy to clean.

In other embodiments, referring to FIG. 3, the bottom wall of the casing 40 is ventilated. The bottom wall of the casing 40 defines a second through hole 401, such that outside air can enter the substrate section 10 through the second through hole 401 and carry the generated aerosol out, which is conducive to reducing the suction resistance of the aerosol-generating product 100.

Specifically, in the aerosol-generating product 100 provided by the present disclosure, a filtering member 30 is arranged in the first airflow channel 21 instead of a filter section. The aerosol-generating product 100 has a two-stage structure, which is simpler in structure and has both cooling and filtering functions, and has low processing costs. In addition, the filtering member 30 is arranged in the first airflow channel 21, which, compared to the conventional aerosol-generating product 100 including a filter section, enables the diameter of the filtering member 30 of the present disclosure to be less, thereby reducing the adsorption effect on the aerosol, increasing the amount of aerosol flowing out from the first airflow channel 21, and thus increasing the amount of aerosol available to the user. Furthermore, the filtering member 30 is arranged in the first airflow channel 21, such that the filtering member 30 can disturb the air flow of the aerosol in the first airflow channel 21, thereby increasing the contact between the aerosol in the first channel and the cooling section 20, which is beneficial for improving the cooling effect.

In some embodiments, referring to FIG. 3, the filtering member 30 includes a second airflow channel 31 that is in fluid communication with the first airflow channel 21 and is configured to divert the aerosol from a side of the filtering member 30 close to the substrate section 10 to the other side. During the diversion process, the filtering member 30 disturbs, adsorbs, and filters the aerosol. The second airflow channel 31 may be a gap within the material of the filtering member 30, or the second airflow channel 31 may be a through hole defined on the filtering member 30.

In other embodiments, the filtering member 30 cooperates with a side wall of the first airflow channel 21 to form a second airflow channel 31 that is in fluid communication with the first airflow channel 21.

For example, referring to FIG. 4, the outer surface of the filtering member 30 defines multiple first grooves 32, an inner wall surface of the first airflow channel 21 a smooth, and the multiple first grooves 32 cooperate with the inner wall surface of the first airflow channel 21 to form the second airflow channel 31.

For another example, referring to FIG. 5, the inner wall surface of the first airflow channel 21 defines multiple second grooves 22 facing the filtering member 30, the outer surface of the filtering member 30 is smooth, and the multiple second grooves 22 cooperate with the outer surface of the filtering member 30 to form the second airflow channel 31.

For further another example, the outer surface of the filtering member 30 defines multiple first grooves 32, and the inner wall surface of the first airflow channel 21 defines multiple second grooves 22. The multiple first grooves 32 corresponds to the multiple second grooves 22 in a one-to-one correspondence, so as to form the second airflow channel 31.

In the present disclosure, the number of first grooves 32 and/or second grooves 22 is at least one, and may be multiple; the depth of each first groove 32 and/or second groove 22 is 0.05 mm-1 mm, as long as the flow rate of the second airflow channel 31 can meet the needs of the user when the user inhales. The specific settings are made according to needs, and are not limited here.

In particular, the first groove 32 and/or the second groove 22 may be arranged in a straight line extending along the axial direction X of the aerosol-generating product 100, i.e., the second airflow channel 31 formed is a through hole, which can reduce the suction resistance.

The first groove 32 and/or the second groove 22 may be arranged along the axial direction X of the aerosol-generating product 100 or in a curved or bent extension relative to the axial direction X, so as to achieve a better turbulence effect on the aerosol.

The shape of the cross-section of the first groove 32 and/or the second groove 22 may be a triangle, arc, polygon, etc., such that the formed second airflow channel 31 can be a triangle, arc, polygon, etc., to meet individual needs, which is not limited herein.

Specifically, the formation method of the second airflow channel 31 is not limited herein, as long as it can have the effect of guiding the aerosol.

In the present disclosure, referring to FIG. 3, in order to achieve a better effect of aggregating the aerosol, the distance H between the substrate section 10 and the filtering member 30 is set to 10 mm-40 mm. It can be understood that if the distance H between the substrate section 10 and the filtering member 30 is too small, the space for the agglomeration of the aerosol formed between the substrate section 10 and the filtering member 30 is too small, the amount of agglomerated aerosol is small, which is difficult to meet the user's needs, and to a certain extent will inhibit the generation of aerosols. If the distance H between the substrate section 10 and the filtering member 30 is too large, the size design of the entire aerosol-generating product 100 will increase, which increases the cost. In addition, the too large distance H may increase the suction force when the user inhales, which affects the usage experience.

In some embodiments, referring to FIG. 2, an end of the filtering member 30 away from the substrate section 10 is flush with an end of the cooling section 20 away from the substrate section 10, which may ensure that there is a space between the substrate section 10 and the filtering member 30 of a certain size for agglomeration of the aerosol, and minimize the size of the aerosol-generating product 100, thereby reducing costs.

Of course, in other embodiments, referring to FIG. 6, the end of the filtering member 30 away from the substrate section 10 may be lower than the end of the cooling section 20 away from the substrate section 10. The related design may be adjusted according to actual needs, as long as the end of the filtering member 30 away from the substrate section 10 is not higher than the end of the cooling section 20 away from the substrate section 10, in order to avoid affecting the appearance of the aerosol-generating product 100 and the filtering effect on the aerosol.

In some embodiments, referring to FIG. 3, in order to ensure that the cooling section 20 can achieve the cooling effect on the aerosol in the first airflow channel 21, the thickness L of the side wall of the first airflow channel 21 is set to 0.5 mm-2.5 mm, which may ensure that the side wall of the first airflow channel 21 has a certain heat absorption capacity. In addition, the outer diameter R of the cooling section 20 is set to 5mm-8mm. The outer diameter R of the cooling section 20 minus the thickness L of the side wall of the first airflow channel 21 will be equal to the aperture diameter of the first airflow channel 21. The above design may be set according to the actual situation.

In the present disclosure, to avoid the temperature of the cooling section 20 being too high after the aerosol is cooled and exchanged heat by the cooling section 20, which might lead to the heat being transmitted to the user through the casing 40, resulting in a poor user experience, the outer surface of the cooling section 20 defines multiple third grooves 23, referring to FIGS. 6-8. The casing 40 surrounds the outer surface of the cooling section 20 and cooperates with the multiple third grooves 23 to form a third airflow channel 24. The casing 40 defines multiple first through holes 42 facing and in communication with the third grooves 23, and the first through holes 42 are configured to guide outside air into the third airflow channel 24. The hot air in the third airflow channel 24 is then sucked in by the user to dissipate heat from the cooling section 20.

Alternatively, the inner surface of the side wall of the casing 40 may define multiple fourth grooves (not shown); the casing 40 surrounds the outer surface of the cooling section 20 and cooperates with the outer surface of the cooling section 20 to form the third airflow channel 24, and the casing 40 defines multiple first through holes 42 facing and in communication with the fourth grooves.

The multiple third grooves 23 and/or fourth grooves are each arranged in a straight-line extension or in a curved extension along the axial direction X of the aerosol-generating product 100. This is not limited, as long as the third airflow channel 24 can be formed and the air flowing in the third airflow channel 24 can take away the heat of the cooling section 20.

In some embodiments, referring to FIG. 6, the third airflow channel 24 includes an open end 25 at one end away from the substrate section 10. When the user inhales the aerosol-generating product 100, outside air enters the third airflow channel 24 through the first through hole 42 and carries the heat from the cooling section 20 out of the open end 25.

In other embodiments, referring to FIG. 7, the third groove 23 extends to an end of the cooling section 20 close to the substrate section 10. When the user inhales the aerosol-generating product 100, the outside air enters the third airflow channel 24 through the first through hole 42, then enters the substrate section 10 connected to the third airflow channel 24, and further follows the generated aerosol through the first airflow channel 21 and the filtering member 30 that is in fluid communication with the first airflow channel 21.

Unlike the related art, the aerosol-generating product 100 provided by the present disclosure has the following advantages. First, the aerosol-generating product 100 has a two-stage structure, which is simpler in structure and has both cooling and filtering functions, with low processing costs. Second, the filtering member 30 is arranged in the first airflow channel 21. Compared with the conventional aerosol-generating product 100 including a filter section, the diameter of the filtering member 30 of the present disclosure is less, which may reduce the adsorption effect on the aerosol, thereby increasing the amount of aerosol flowing out from the first airflow channel 21, and thus increasing the amount of aerosol available to the user. Third, the filtering member 30 is arranged in the first airflow channel 21, and the filtering member 30 can disturb the airflow of the aerosol in the first airflow channel 21, thereby increasing the contact between the aerosol in the first airflow channel and the cooling section 20, which is conducive to improving the cooling effect. Fourth, the outer surface of the cooling section 20 cooperates with the casing 40 to form a third airflow channel 24, and the casing 40 defines multiple first through holes 42 corresponding to the position of the third airflow channel 24 and communicating with the third airflow channel 24. When the user inhales the aerosol-generating product 100, the outside air enters the third airflow channel 24 through the first through hole 42 to dissipate heat from the cooling section 20, and the hot air in the third airflow channel 24 is sucked out by the user to dissipate heat from the cooling section 20.

Referring to FIG. 9, FIG. 9 is a structural schematic view of an aerosol-generating system according to some embodiments of the present disclosure. The present disclosure further provides an aerosol-generating system 300, including an aerosol-generating product 100 and an aerosol-generating device 200. The aerosol-generating device 200 includes a receiving cavity (not shown) and a heating component (not shown), where the receiving cavity is configured to contain the aerosol-generating product 100, and the heating component is configured to heat the aerosol-generating product 100 to generate an aerosol. The aerosol-generating product 100 may be any of the aerosol-generating products provided in any of the above embodiments.

The above is only some embodiments of the present disclosure, and does not thereby limit the scope of the present disclosure. Any equivalent structure or equivalent process transformation using the content of the description and drawings of the present disclosure, or direct or indirect use in other related technical fields, shall likewise be included in the scope of the present disclosure.

## Claims

1. An aerosol-generating product, comprising:
a substrate section, arranged with an aerosol-generating substrate;
a cooling section, disposed on an end of the substrate section and comprising a first airflow channel; and
a filtering member, disposed in the first airflow channel;
wherein the filtering member comprises a second airflow channel; and/or, the filtering member cooperates with the side wall of the first airflow channel to form a second airflow channel; the second airflow channel is in fluid communication with the first airflow channel.

2. The aerosol-generating product according to claim 1, wherein the outer surface of the filtering member defines a plurality of first grooves, and the plurality of first grooves cooperate with the inner wall surface of the first airflow channel to form the second airflow channel; or,
the inner wall surface of the first airflow channel defines a plurality of second grooves facing the filtering member, and the plurality of second grooves cooperate with the outer surface of the filtering member to form the second airflow channel; or,
the outer surface of the filtering member defines a plurality of first grooves, and the inner wall surface of the first airflow channel defines a plurality of second grooves; the plurality of first grooves cooperate with the plurality of second grooves to form the second airflow channel.

3. The aerosol-generating product according to claim 2, wherein each first groove and/or each second groove is arranged in a straight-line extension or in a curved extension along the axial direction of the aerosol-generating product.

4. The aerosol-generating product according to claim 1, wherein the distance between the substrate section and the filtering member is in a range of 10 mm-40 mm.

5. The aerosol-generating product according to claim 1, wherein the end of the filtering member away from the substrate section is flush with the end of the cooling section away from the substrate section.

6. The aerosol-generating product according to claim 1, further comprising:
a casing, arranged on the outer surfaces of the substrate section and the cooling section.

7. The aerosol-generating product according to claim 6, wherein the outer surface of the cooling section defines a plurality of third grooves; the casing cooperates with the plurality of third grooves to form a third airflow channel; the casing defines a plurality of first through holes facing and in communication with the plurality of third grooves, and the plurality of first through holes are configured to guide outside air into the third airflow channel.

8. The aerosol-generating product according to claim 7, wherein each third groove is arranged in a straight-line extension or in a curved extension along the axial direction of the aerosol-generating product.

9. The aerosol-generating product according to claim 7, wherein the end of the third airflow channel away from the substrate section is an open end; the outside air is capable of entering the third airflow channel through the plurality of first through holes and flowing out of the open end.

10. The aerosol-generating product according to claim 7, wherein each third groove extends to the end of the cooling section close to the substrate section; the outside air is capable of entering the third airflow channel through the plurality of first through holes, then entering the substrate section connected to the third airflow channel, and flowing out through the first airflow channel and the second airflow channel.

11. The aerosol-generating product according to claim 6, wherein the casing comprises sidewalls arranged on side surfaces of the substrate section and the cooling section, and a bottom wall arranged on the end of the substrate section away from the cooling section; the bottom wall defining a second through hole.

12. An aerosol-generating system, comprising:
the aerosol-generating product according to any one of claims 1-11; and
an aerosol-generating device, comprising a receiving cavity and a heating component; wherein the receiving cavity is configured to contain the aerosol-generating product, and the heating component is configured to heat the aerosol-generating product to generate an aerosol.
